# EUROPEAN PATENT SPECIFICATION

(11) **EP 4 568 455 B1**
(45) Date of publication and mention of the grant of the patent: **22.07.2026**
(21) Application number: 24213601.8
(22) Date of filing: 18.11.2024
(51) Int. Cl.: H10H 29/14, H10H 29/30

(54) **ELECTRODE SYSTEM AND COMPLEMENTARY METAL-OXIDE-SEMICONDUCTOR-BASED DEVICE INCLUDING THE SAME**
ELEKTRODENSYSTEM UND KOMPLEMENTÄRE METALLOXIDHALBLEITERBASIERTE VORRICHTUNG DAMIT
SYSTÈME D'ÉLECTRODE ET DISPOSITIF À BASE DE SEMI-CONDUCTEUR À OXYDE MÉTALLIQUE COMPLÉMENTAIRE LE COMPRENANT

(30) Priority: 08.12.2023 KR 20230177790
(43) Date of publication of application: 11.06.2025
(73) Proprietor: Samsung Electronics Co., Ltd., Suwon-si, Gyeonggi-do 16677 (KR)
(72) Inventor: de Jesus Alcantara, Carlos Cristiano, 16678 Suwon-si, Gyeonggi-do (KR); Ju, Anes, 16678 Suwon-si, Gyeonggi-do (KR); Hong, Hyeokki, 16678 Suwon-si, Gyeonggi-do (KR); Park, Kitae, 16678 Suwon-si, Gyeonggi-do (KR); Bae, Chisung, 16678 Suwon-si, Gyeonggi-do (KR)
(74) Representative: Grünecker Patent- und Rechtsanwälte PartG mbB

(56) References cited:
- KR-B1- 101 681 174
- US-A1- 2017 122 928

## Description

### BACKGROUND

### 1. Field

Methods and apparatuses consistent with embodiments of the disclosure relate to an electrode system, and a complementary metal-oxide-semiconductor (CMOS)-based device including the same.

### 2. Description of Related Art

Recently, various machine learning approaches are implemented based on neural networks. However, such machine learning approaches may not actually mimic the brain. Therefore, in order to actually mimic the brain, various energy-efficient computing devices and systems are being proposed to mimic the neuronal systems (e.g., single neurons, two-dimensional (2D) neuronal networks, brain organoids, etc.). Considering the complexity of the human brain, one of the more technically feasible approach for computer engineering purposes is to study simpler systems, such as *in-vitro* 2D neuronal systems.

One related art technique in electrophysiology involves recording the intracellular activity of neurons is a patch-clamp technique, where a probe is inserted in preselected regions of neurons (i.e., somas, axons, dendrites, etc.) through which the flow of electric charge can be externally modulated to excite and/or record a neuronal activity.

However, due to the operational complexity and bulkiness of the probe, patch-clamp experiments are inherently slow and only a few dozen channels can be recorded simultaneously.

A considerably higher experimental throughput is required for patch-clamp recording, which makes the patch-clamp recording unfeasible for the purpose of computer development. To this end, complementary metal-oxide-semiconductor (CMOS)-based multi-electrode arrays (MEAs) have been developed. For example, a related art 2D-MEAs may be used for large-scale neuronal recordings. However, the performance of 2D-MEAs is still sub-par compared to patch clamp methods. The document KR 101681174 B1 discloses an photo active chip which can form conductive pathways for cell measurements.

### SUMMARY

One or more embodiments may address at least the above problems and/or disadvantages and other disadvantages not described above. Also, the embodiments are not required to overcome the disadvantages described above, and an embodiment may not overcome any of the problems described above.

According to an aspect of the disclosure, there is provided an electrode system including: a light-emitting diode (LED) display layer comprising a single LED layer or a plurality of LED layers; a first photoconductive layer on the LED display layer; a second photoconductive layer on the first photoconductive layer; and an electrode layer provided on the LED display layer, the first photoconductive layer or the second photoconductive layer.

The LED display layer may include a nano-LED or a micro-LED.

An LED in the LED display layer may include at least one of a group II-VI compound semiconductor, a group III-V compound semiconductor or a group I-III-VI compound semiconductor, or a combination thereof.

The LED display layer may include a plurality of LEDs, wherein the plurality of LEDs may be configured to form a plurality of pixel units based on a single LED, among the plurality of LEDs, or based on an arrangement of a plurality of LEDs, wherein the plurality of LEDs may be configured to adjust a light emitting region, a shape of the light emitting region, or a light emission intensity within the plurality of pixel units, and wherein a spacing between the plurality of pixel units may be empty or filled with a photoconductive material.

The electrode system may further include: a nano-positioner or a micro-positioner configured to independently shift horizontally or vertically, or rotate one or more of the plurality of LED layers, which are stacked.

The nano-positioner or the micro-positioner may be further configured to: shift the one or more of the plurality of LED layers such that positions of pixel units of neighboring LED layers overlap in a range of 0% to 100%; or shift the one or more of the plurality of LED layers such that light emitted from pixel units of neighboring LED layers overlap.

A first surface roughness of the first photoconductive layer may be different from a second surface roughness of the second photoconductive layer.

The first photoconductive layer may include a flat film surface, and the second photoconductive layer may include a textured surface with a three-dimensional (3D) structure including at least one of a spherical shape, an oval shape, a conical shape, a polygonal pyramidal shape, a polygonal pillar shape, a polygonal shape, or a star polygonal shape, or a combination thereof.

Each of the first photoconductive layer and the second photoconductive layer may include at least one of a photoconductive polymer, a photoconductive chalcogenide, a photoconductive oxide or a photoconductive amorphous silicon-based material, or a combination thereof.

The electrode layer may include a plurality of electrode patterns on a support substrate, and each of the of the plurality of electrode patterns may include at least one of a metal, a conductive oxide or a conductive polymer, or a combination thereof.

The electrode layer may include a plurality of electrode patterns that is at least partially inserted in a depth direction of a support substrate and each of the plurality of electrode patterns has an exposed top surface.

The support substrate may be the first photoconductive layer.

The support substrate may be a transparent substrate or opaque substrate.

A distance between the LED display layer and the second photoconductive layer may be in a range of 1 nm to 1,000 nm.

The electrode system may be a monolithic integration system in which the electrode layer is provided on a lower region of the LED display layer, and the electrode layer may include: a support substrate; and an electrode pattern layer on the support substrate, and wherein the LED display layer may be in contact with the electrode pattern layer.

The electrode system may be a heterogeneous integration system in which the electrode layer is provided on an upper region of the LED display layer and on the second photoconductive layer, and wherein each of the plurality of LED layers may include a nano-positioner or a micro-positioner.

The electrode system may be configured to perform an imaging, a stimulation or a signal detection of a cell in vitro or in vivo, and the electrode system may include a light-addressable electrode provided in the electrode layer, the light-addressable electrode configured to: set a position or a range of a sample based on at least one of a position of light emitted from the LED display layer, an area of light, a shape of a light spot or a light intensity, and reconfigure a position or a size of the light-addressable electrode.

According to another aspect of the disclosure, there is provided a complementary metal-oxide-semiconductor (CMOS)-based device including: a CMOS chip; and an electrode system including: a light-emitting diode (LED) display layer including a single LED layer or a plurality of LED layers; a first photoconductive layer on the LED display layer; a second photoconductive layer on the first photoconductive layer; and an electrode layer provided on the LED display layer, the first photoconductive layer or the second photoconductive layer, wherein the CMOS chip and the electrode system are integrated.

The CMOS-based device may further include a bias configured to provide a light source to an upper end, a lower end, or both the upper end and the lower end of the electrode system, and the electrode system may be monolithically or heterogeneously integrated on the CMOS chip.

The CMOS-based device may be an electrophysiological system-on-a-chip (SOC) for recording or stimulating a signal from a neuron, or a CMOS-based cell recording device used to identify a neuronal system and evaluate a neuronal-based algorithm with a neuron in a loop.

### BRIEF DESCRIPTION OF DRAWINGS

The above and/or other aspects will be more apparent by describing certain embodiments with reference to the accompanying drawings, in which:
FIG. 1 is a cross-sectional view illustrating a configuration of an electrode system according to one or more embodiments;
FIG. 2A is a cross-sectional view illustrating an example of the electrode system of FIG. 1 in which light-emitting diode (LED) display layers are monolithically integrated;
FIG. 2B is a cross-sectional view illustrating another example of the electrode system of FIG. 1 in which the LED display layers are heterogeneously integrated;
FIG. 3A illustrates an example of providing a light source by an external bias according to one or more embodiments;
FIG. 3B illustrates an example of reaching a high conductive threshold by the external bias shown in FIG. 3A and implementing a continuous conductive path from discrete light sources, according to one or more embodiments;
FIGS. 4A to 4E illustrate a two-dimensional (2D) patterning procedure using a light-addressable electrode based on an LED display of an electrode system, according to one or more embodiments;
FIG. 5 is a flowchart illustrating a method of identifying a neuronal network or a neuronal system using a light-addressable electrode based on an LED display of an electrode system, according to one or more embodiments; and
FIG. 6 is a flowchart illustrating a method of evaluating a neuronal-based algorithm with a neuron-in-the-loop and a light-addressable electrode based on an LED display of an electrode system, according to one or more embodiments.

### DETAILED DESCRIPTION

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular example embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. Terms, such as those defined in commonly used dictionaries, are to be interpreted as having a meaning that is consistent with their meaning in the context of the relevant art and the disclosure, and are not to be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the disclosure.

In addition, terms such as first, second, A, B, (a), (b), and the like may be used to describe components of the embodiments. Each of these terms is not used to define an essence, order or sequence of a corresponding component but used merely to distinguish the corresponding component from other component(s). It should be noted that if it is described in the specification that one component is "connected," "coupled" or "joined" to another component, the former may be directly "connected", "coupled", and "joined" to the latter or "connected", "coupled", and "joined" to the latter via another component.

As used herein, "A or B", "at least one of A and B", "at least one of A or B", "A, B or C", "at least one of A, B and C", and "at least one of A, B, or C", each of which may include any one of the items listed together in the corresponding one of the phrases, or all possible combinations thereof.

A component, which has the same common function as a component included in any one embodiment, will be described by using the same name in other embodiments. Unless otherwise mentioned, the descriptions on the embodiments may be applicable to the following embodiments and thus, duplicated descriptions will be omitted for conciseness.

Hereinafter, embodiments will be described in detail with reference to the accompanying drawings. When describing the embodiments with reference to the accompanying drawings, like reference numerals refer to like components and a repeated description related thereto will be omitted.

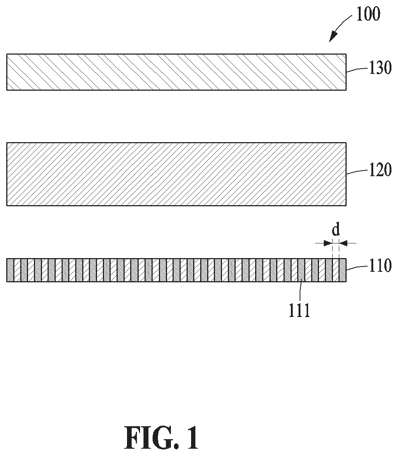

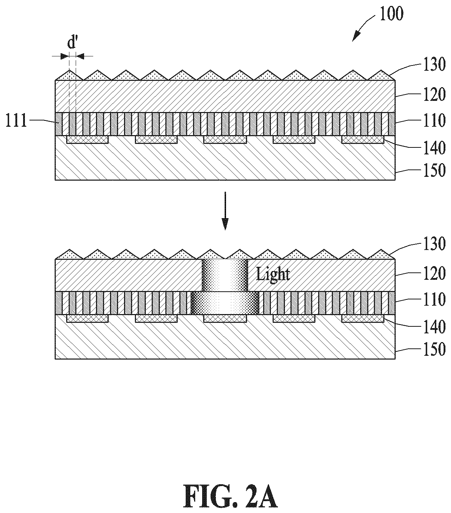

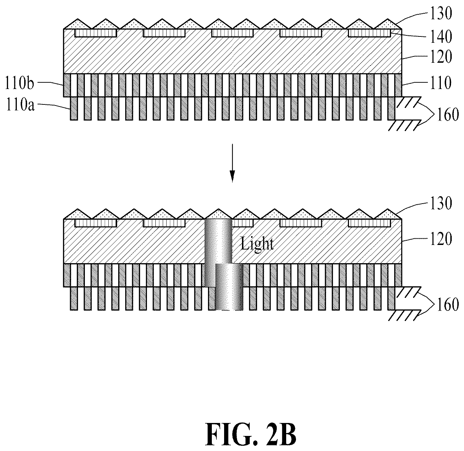

FIG. 1 is a cross-sectional view of an electrode system 100 according to one or more embodiments. FIG. 2A is a cross-sectional view of the electrode system 100 in which light-emitting diode (LED) display layers are monolithically integrated. FIG. 2B is a cross-sectional view of the electrode system 100 in which LED display layers are heterogeneously integrated.

Referring to FIG. 1, FIG. 2A and FIG. 2B, according to an embodiment, the electrode system 100 may include an LED display layer 110, a first photoconductive layer 120, a second photoconductive layer 130, and an electrode layer 140.

According to an embodiment, the electrode system 100 may implement reconfigurable electrodes. For example, the reconfigurable electrodes may reconfigure an electrode distribution using the LED display layer 110. For example, the reconfigurable electrodes may reconfigure a spatial distribution of electrodes. According to an embodiment, the reconfigurable electrodes may include one or more light-addressable electrodes.

According to an embodiment, the LED display layer 110 may include a nano-LED or a micro-LED. In some examples, the LED display layer 110 may provide a nanometer-scale resolution or micrometer-scale resolution. In some examples, the LED display layer 110 may provide an addressable area in a nanometer range, a micrometer range, a millimeter range, or a meter range. Here, the addressable area may be a scalable addressable area and/or a light-addressable area. In some examples, the LED display layer 110 may implement reconfigurable electrodes that may reconfigure an electrode distribution with the nanometer-scale resolution or micrometer-scale resolution through light addressing.

According to an embodiment, the LED display layer 110 may include a single LED, or a plurality of LEDs. In some examples, the plurality of LEDs may be regularly arranged or distributed. In some other examples, the plurality of LEDs may be irregularly arranged or distributed. In some examples, pixel units 111 with various shapes may be formed depending on an arrangement or distribution method of the plurality of LEDs. For example, the pixel units 111 may be LED cells.

According to an embodiment, the LED display layer 110 may form a plurality of pixel units by patterning of an LED (e.g., patterning of an LED film) or an arrangement of the plurality of LEDs. As such, in some example, the pixel units 111 may be referred to as pattern units. Through the above patterning of the LED, a stereoscopic structure (e.g., a three-dimensional (3D) or 2D structure) may be formed by etching a flat LED film and predetermined areas may be compartmentalized. Accordingly, a plurality of pixel units (e.g., LED cells) may be formed by a single LED. In some examples, one LED among the plurality of LEDs may be one pixel unit 111 among the plurality of pixel units.

According to an embodiment, the resolution of the electrode distribution may be adjusted depending on a size of an LED or a size of a pixel unit 111 (e.g., an LED cell). For example, the size of the pixel unit 111 may be an area, a width, or a length (d') as shown in FIG. 2A. For example, an LED or a pixel unit (e.g., an LED cell) may have a size of 1 nanometer (nm) to less than 1000 micrometers (µm); 10 nm to 900 µm; 20 nm to 500 µm; or 20 nm to 200 µm. In some examples, a large area addressability independent from the resolution may be provided.

According to an embodiment, the LED display layer 110 may individually control LEDs or pixel units (e.g., LED cells).

According to an embodiment, the LED display layer 110 may adjust at least one of a light emitting region, a shape of the light emitting region, and a light emission intensity within the plurality of pixel units, and may provide light patterning or setting of an addressable area (e.g., a light-addressable area) and a resolution. However, the disclosure is not limited thereto, and as such, according to an embodiment, the LED display layer 110 may adjust two or more of the light emitting region, the shape of the light emitting region, and the light emission intensity within the plurality of pixel units to provide light patterning or setting of the addressable area (e.g., a light-addressable area) and the resolution. In this manner, the LED display layer 110 may selectively reconfigure the reconfigurable electrodes. Thus, reconfigurable electrodes capable of providing a distribution with a nanometer-scale resolution or micrometer-scale resolution while having an addressable area in the nanometer range to the millimeter range may be realized. The above reconfigurable electrodes may be spatially reconfigured.

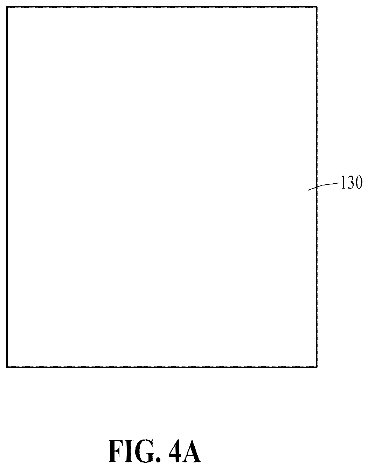

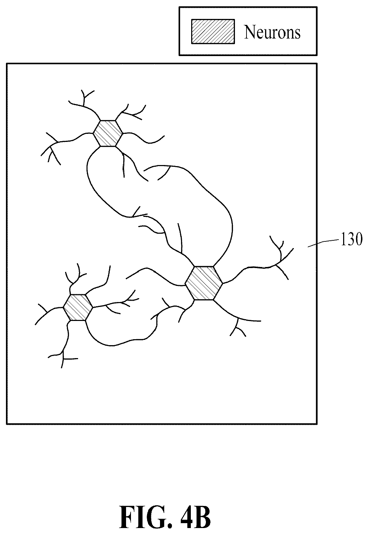

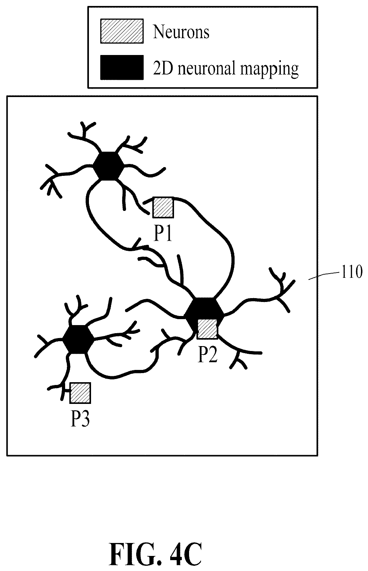

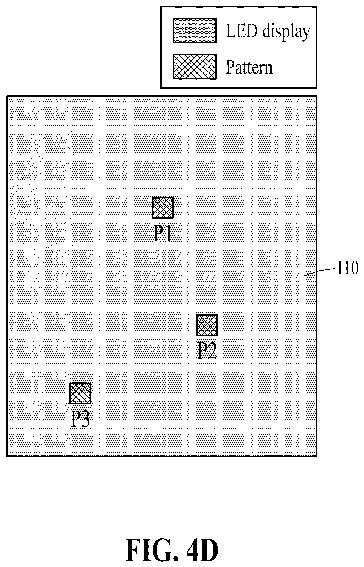

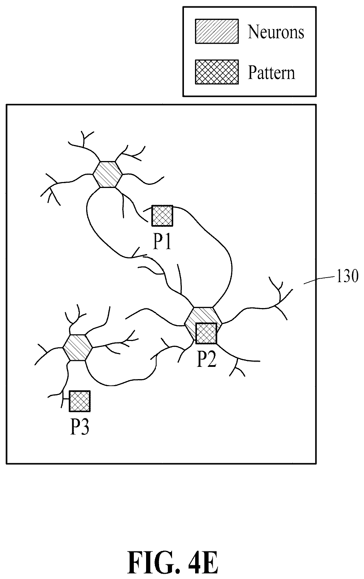

According to an embodiment, FIGS. 4A to 4E illustrate a 2D patterning procedure using a light-addressable electrode based on an LED display of an electrode system. Referring to FIG. 4A, cells may be grown on the top of a bare photoconductive thin film. For example, neuronal cells (including somas, axons, dendrites, etc.) may be grown on a second photoconductive thin film layer 130 shown in FIG. 1. Referring to FIG. 4B, a neuron network formed by the cell growth may be imaged and a position of a neuron may be mapped based on the image. For example, the neuron network may be imaged using an optical microscopy. For example, the mapping of the position of the neuron may be performed using a stereoscopic microscopy or a fluorescence microscopy. Referring to FIG. 4C, points of interest may be manually selected through a graphical user interface (GUI) or may be selected as a portion of an automated procedure. For example, points of interests P1, P2 and P3 may be manually selected thought the GUI. However, the disclosure is not limited thereto, and as such, according to another embodiment, the points of interests P1, P2 and P3 may be specified by an automated procedure, such as, but not limited to a feedback loop. Referring to FIG. 4D, in an example case in which points of interest P1, P2, and P3 are selected or specified, a corresponding pattern is transmitted to a micro-LED display 110 or nano-LED display 110. For example, light patterning may be performed on the points of interest P1, P2, and P3. For example, a 2D patterning corresponding to the mapping of the position of the neuron in the points of interest P1, P2, and P3 may the performed on the LED display 110. Referring to FIG. 4E, reconfigurable electrodes may be configured for stimulation and recording for the points of interest P1, P2, and P3.

Referring to FIG. 4C, the LED display layer 110 may individually control an intensity of pixel units (e.g., LED cells) or LEDs according to an embodiment, and as such, discretely spaced light-addressable electrodes or a continuous path may be realized. However, the disclosure is not limited thereto, and as such, according to another embodiment, the LED display layer 110 may control intensity of pixel units (e.g., LED cells) or LEDs in a combined manner. For example, the intensity of two or more pixel unites may be controlled together.

According to an embodiment, in the LED display layer 110, a plurality of LEDs or a plurality of pixel units may be spaced apart from each other. Referring to FIG. 1, a spacing (d) between the LEDs or a spacing between the pixel units 111 may include an electrically or optically inactive region or may be coated or filled with a material for implementing an electrically or optically inactive region. For example, the inactive region may be a physically or chemically treated region. In some examples, the above spacing may be empty or may include or be filled with a photoconductive material. For example, the spacing (d) may include the same material as the first photoconductive layer 120 or the second photoconductive layer 130. However, the disclosure is not limited thereto, and as such, the spacing (d) may include another material or an empty space.

According to an embodiment, an LED in the LED display layer 110 may include a semiconductor material for implementing an LED with a 2D structure, a 3D structure, or a flat film. In some examples, the LED may include at least one of a group II-VI compound semiconductor, a group III-V compound semiconductor, or a group I-III-VI compound semiconductor, or a combination thereof, but is not limited thereto. As such, according to another embodiment, the LED may include another material. In some examples, to implement an LED display, a basic configuration and a material for light emission may use a configuration known in the art to which the disclosure belongs and are not described in detail herein.

According to an embodiment, the LED display layer 110 may be a single layer, or a plurality of layers that is stacked. In an example case in which a plurality of layers is stacked, the layers may be the same or different from each other.

In an example case in which a plurality of LED display layers 110 are stacked, each of the LED display layers 110 may further include a nano-positioner or a micro-positioner 160 as shown in FIG. 2B. In some examples, a positioner 160 may independently shift or rotate each of the LED display layers 110. In some examples, the positioner 160 may independently shift each of the LED display layers 110 vertically or horizontally (e.g., forward and backward, or leftward and rightward). However, the disclosure is not limited thereto, and as such, the positioner 160 may shift or rotate two or more of the LED display layers 110 simultaneously.

In an example case in which a plurality of LED display layers 110 are stacked, the plurality of LED display layers 110 may be adjusted such that positions of LEDs or pixel units of neighboring LED display layers 110 may overlap in a range of about 0% to about 100%; about 0% to less than about 100%; about 0% to about 90%; about 10% to about 60%; or about 20% to about 40%. Here, "%" may indicate an overlap range of an area, a length, a width, and the like, of the above positions, which may be measured based on a vertical direction (i.e., a height direction) (e.g., a Y-axis direction).

According to an embodiment, light beam forming based on multiple micro/nano-LED displays may be provided using a micro-positioner and a nano-positioner. For example, a mixed LED display device including a micro LED display and a nano LED display may be provided. For example, the mixed LED display device may be configured to emit both micro-scale resolution light and nano-scale resolution light. In some cases, the mixed LED display device may be referred to as the multiple micro/nano-LED displays.

According to an embodiment, at least one of a position of a light emitting region, a size of a light emitting region, a shape of a light emitting region (e.g., a light spot) or the light emission intensity within a plurality of pixel units, or a combination thereof may be adjusted through adjustment of a spatial arrangement of the LED display layers 110, and a nanometer-scale resolution or micrometer-scale resolution or an addressable area (e.g., a light-addressable area) may be provided. Accordingly, reconfigurable electrodes (e.g., light-addressable electrodes) that may reconfigure a distribution with the nanometer-scale resolution or micrometer-scale resolution while having an addressable area in the nanometer to millimeter range may be implemented. The above reconfigurable electrodes may be spatially reconfigured.

According to an embodiment, the LED display layer 110 may have a thickness of about 1 nm (nanometer) to about 1,000 *µ*m (micrometer); about 10 nm (nanometer) to about 800 *µ*m (micrometer); about 100 nm (nanometer) to about 500 *µ*m (micrometer); or about 200 nm (nanometer) to about 100 *µ*m (micrometer). In some examples, the thickness may be a thickness of a single LED display layer 110 or all the LED display layers 110 combined.

According to an embodiment, the LED display layer 110 may be an LED display with a small area or a large area. In some examples, the LED display layer 110 may have an area in a range of 10 inches or greater; in a range of 20 inches or greater; in a range of 30 inches or greater; in a range of 50 inches or greater; in a range of 80 inches or greater; or in a range of 100 inches or greater.

According to an embodiment, the LED display 100 may be a front projection system or a rear projection system.

According to an embodiment, the first photoconductive layer 120 may be provided on the LED display layer 110, and the second photoconductive layer 130 may be provided on the first photoconductive layer 120. According to an embodiment, the first photoconductive layer 120 may have the same or different components as the second photoconductive layer 130. According to an embodiment, the first photoconductive layer 120 may have the same or different thickness as the second photoconductive layer 130. In some examples, the first photoconductive layer 120 and the second photoconductive layer 130 may be designed to have different surface characteristics (e.g., a surface roughness) depending on components of the first photoconductive layer 120 and the second photoconductive layer 130. In some examples, the first photoconductive layer 120 and the second photoconductive layer 130 may each include a photoconductive material that includes at least one of a photoconductive polymer, a photoconductive chalcogenide (e.g., molybdenum disulfide), a photoconductive oxide (e.g., TiO₂) or a photoconductive amorphous silicon-based material (e.g., a-Si:H), or a combination thereof.

In some examples, the photoconductive polymer may include at least one of polyvinylcarbazole (PVK), polysiloxane carbazole, polyparaphenylenevinylene, polyaniline, polypyrrole, polyacetylene, polythiophene, polyalkylthiophene, carbazole-substituted polysiloxane (PSX-Cz), poly(p-phenylene terephthalate) carbazole (PPT-CZ), polyacrylate triphenylamine (TATPD), derivatives or copolymers thereof, or a combination thereof. In some examples, the photoconductive oxide may be a metal oxide with photoconductive properties, and may include at least one of CuO, CuO₂, NiO, ZnO, Ag₂O, MnO, TiO₂, BaO, PbO, CeO₂, Bi₂O₃, CdO, Fe₃O₄, or perovskite oxide, or a combination thereof. In some examples, the photoconductive chalcogenide may include at least one of sulfide chalcogenide, selenide chalcogenide or telluride chalcogenide, or a combination thereof. In some examples, a chalcogenide may include at least one of CuInS₂, ZnSSe, CuCr₂S₄, InGaAs, PbS, CdS, ZnS, Ag₂S, MnS, Bi₂S₃, Sb₂S₃, As₂S₃, SnS, SnS₂, In₂S₃, CuS, Cu₂S, CoS, NiS, MoS₂, FeS₂, CrS₃, PbSe, CdSe, ZnSe, Bi₂Se₃, HgSe, Sb₂Se₃, As₂Se₃, NiSe, TlSe, CuSe, Cu₂Se, MoSe₂, SnSe, CoSe, In₂Se₃, PbTe, CdTe, ZnTe, HgTe, Bi₂Te₃, As₂Te₃, Sb₂Te₃, NiTe, TlTe, CuTe, MoTe₂, SnTe, CoTe, Ag₂Te or In₂Te₃, or a combination thereof.

According to an embodiment, the first photoconductive layer 120 may provide surface characteristics (e.g., a surface roughness) different from those of the second photoconductive layer 130. In some examples, the surface characteristics of the first photoconductive layer 120 may be a flat film surface. In some examples, the second photoconductive layer 130 may include a textured surface. In some examples, the second photoconductive layer 130 may include a textured surface with a 3D structure. In some examples, the 3D structure may be regularly or irregularly arranged and the shape of the 3D structure may include at least one of a spherical shape, an oval shape, a conical shape, a polygonal pyramidal shape, a polygonal pillar shape, a polygonal shape, or a star polygonal shape, or a combination thereof. In some examples, the 3D structure may have a height (e.g., an average height) of about 10 nm (nanometer) to about 10 *µ*m (micrometer); about 50 nm (nanometer) to about 8 *µ*m (micrometer); about 100 nm (nanometer) to about 5 *µ*m (micrometer); about 200 nm (nanometer) to about 2*µ*m (micrometer); or about 300 nm (nanometer) to about 1 *µ*m (micrometer).

According to an embodiment, each of the first photoconductive layer 120 and the second photoconductive layer 130 may have a thickness of about 1 nm (nanometer) to about 1,000 nm (nanometer); about 50 nm (nanometer) to about 800 nm (nanometer); about 100 nm (nanometer) to about 500 nm (nanometer); or about 100 nm (nanometer) to about 200 nm (nanometer).

According to an embodiment, a distance between the LED display layer 110 and the second photoconductive layer 130 may be in a range of about 0 nm (nanometer)(e.g., conformal deposition) to about 1 mm (millimeter)(e.g., heterogeneous integration); about 1 nm (nanometer) to about 1 mm (millimeter); about 100 nm (nanometer) to about 1 mm (millimeter); about 300 nm (nanometer) to about 1 mm (millimeter); about 500 nm (nanometer) to about 1 mm (millimeter); or about 1,000 nm (nanometer) to about 1 mm (millimeter). In some examples, in the above distance, a resolution (e.g., a light dispersion profile) may be controlled or enhanced. In some examples, a precise calibration of light patterning at the distance may be provided.

According to an embodiment, the electrode layer 140 may be provided in a lower region, an upper region, or both the lower region and the upper region of the LED display layer 110. In an example, the electrode layer 140 may be provided between the first photoconductive layer 120 and the second photoconductive layer 130. In another example, the electrode layer 140 may be provided between the first photoconductive layer 120 and the LED display layer 110.

According to an embodiment, the electrode layer 140 may provide a conductive electrode function, and any conductive material may be applicable without limitation. In some examples, the electrode layer 140 may include transparent or opaque conductive materials (conductors). In some examples, the electrode layer 140 may include at least one of a metal, an alloy, an intermetallic compound, a conductive oxide or a conductive polymer, or a combination thereof. In some examples, the metal may include at least one of Cu, Co, Ir, Ta, In, Cr, Mn, Mo, Tc, W, Re, Fe, Sc, Ti, Sn, Ge, Sb, Al, Ag, Pt, Ni, or Au or a combination thereof, but is not limited thereto. In some examples, an oxide, an alloy, and an intermetallic compound may include the above metals. In some examples, the oxide may be, for example, indium tin oxide (ITO), indium gallium oxide (IGO), indium zinc oxide (IZO), indium gallium zinc oxide (IGZO), and the like. In some examples, the oxide may be mixed with a conductive organic material or may include a conductive organic material layer. In some examples, the conductive organic material may include at least one of a carbon nanotube (CNT), graphene, or a conductive polymer. The conductive polymer may include at least one of poly(3,4-ethylenedioxythiophene)/poly(4-styrene sulfonate) (PEDOT/PSS), polyaniline (PANI), polypyrrole (PPy), polythiophene (PT), polyacetylene (PA), poly para-phenylene vinylene (PPV), polyparaphenylene (PPP) or poly sulfur nitride ((SN)x), or a combination thereof, but is not limited thereto.

According to an embodiment, the electrode layer 140 may include a plurality of electrode patterns on a support substrate. According to an embodiment, the plurality of electrode patterns are reconfigurable. In some examples, at least a portion of a height of an electrode pattern may be provided in a depth direction of the support substrate, and a top surface may be exposed. In some examples, at least a portion of a height of an electrode pattern may be inserted or embedded in a depth direction of the support substrate, and a top surface may be exposed. In some examples, the support substrate may be a transparent or opaque substrate. In some examples, as shown in FIG. 2A, as the support substrate 150, any support substrate that may include an element for supporting or driving the electrode layer 140 or that may be compatible with such the element may be applicable without limitation. In some examples, a support substrate may be a substrate that is compatible with a driving substrate (e.g., a semiconductor chip or semiconductor-based substrate) (e.g., a CMOS chip or a CMOS-based substrate). In some examples, the support substrate 150 may be a substrate applicable in vivo or in vitro. In some examples, the support substrate 150 may be a biocompatible polymer, a wafer, glass, silicon, sapphire, a semiconductor substrate, and the like, but is not limited thereto. In some examples, as shown in FIG. 2B, the support substrate may be the first photoconductive layer 120.

According to an embodiment, the electrode system 100 may implement a light-addressable electrode. In some examples, an LED display layer may be used to designate a single position or a plurality of positions on a specimen (e.g., cells, neurons, etc.) and perform a task such as signal detection, measurement, imaging, and stimulation by electrodes at the designated position.

According to an embodiment, referring to FIG. 2A, the electrode system 100 may be a monolithic integration system. The monolithic integration system may be monolithically integrated with a CMOS chip. In some examples, the monolithic integration system may include an LED display layer 110, a first photoconductive layer 120 on the LED display layer 110, and a second photoconductive layer 130 on the first photoconductive layer 120. The monolithic integration system may further include an electrode layer 140 in a lower region of the LED display layer 110. The electrode layer 140 may include a support substrate 150, sand an electrode pattern layer on the support substrate 150. The LED display layer 110 may be in contact with the electrode pattern layer.

According to an embodiment, referring to FIG. 2B, the electrode system 100 may be a heterogeneous integration system. The heterogeneous integration system may be heterogeneously integrated with a CMOS chip. In some examples, the heterogeneous integration system may include an LED display layer 110, a first photoconductive layer 120 on the LED display layer 110, and a second photoconductive layer 130 on the first photoconductive layer 120. The heterogeneous integration system may further include an electrode layer 140 in an upper region of the LED display layer 110. The electrode layer 140 may be provided on the second photoconductive layer 130. A plurality of LED display layers may be provided, and each of the LED display layers may include a nano-positioner or a micro-positioner.

According to an embodiment, in an example case in which a resolution of a positioner is greater than a resolution of the LED display layer 110 in FIG. 2B, a shift may be generated between the respective LED display layers, to obtain a resolution greater than a size of an LED.

According to an embodiment, each of the LED display layers 110 may independently horizontally shift and rotate in FIG. 2B, and accordingly, a desired light pattern may be more accurately tuned. In addition, a curved conductive path may be realized.

According to an embodiment, a CMOS-based device may include a CMOS chip and the electrode system according to embodiments that are integrated. According to an embodiment, the CMOS chip may be a CMOS-based multi-electrode array (MEA). According to an embodiment, the CMOS chip may include a memory and a processor. For example, the memory may store one or more instructions, and the processor may executed the one or more instructions to perform one or more operations of the CMOS chip and/or the electrode system.

According to an embodiment, the CMOS-based device may further include an external bias to provide a light source (e.g., light energy) toward an upper end, a lower end, or both of the electrode system 100. Thus, a light intensity of a single LED cell is reached an energy threshold of a photoconductor required to generate a conductive path through a photoconductive layer.

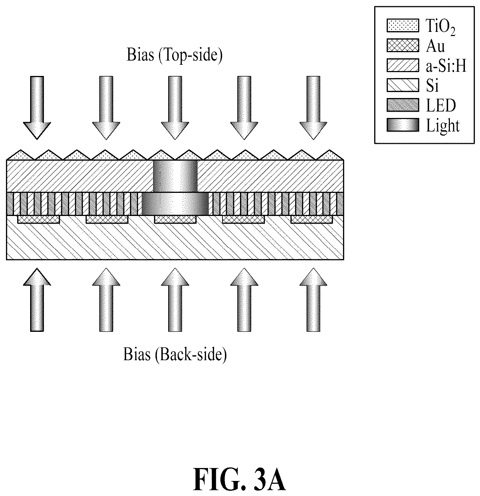

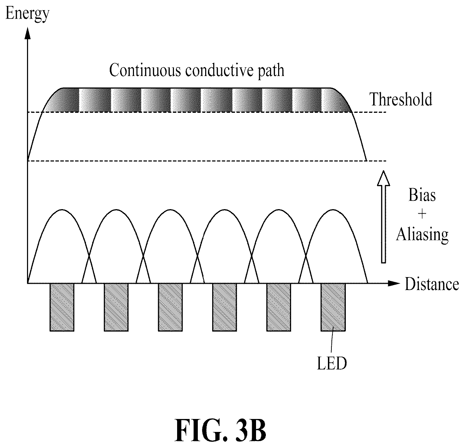

FIG. 3A illustrates an example of providing a light source by an external bias, according to one or more embodiments. FIG. 3B illustrates an example of reaching a high conductive threshold by the external bias shown in FIG. 3A and implementing a continuous conductive path from discrete light sources, according to one or more embodiments. As shown in FIGS. 3A and 3B, the external bias may be a global back-side illumination source or topside illumination source. In some examples, the external bias may use an aliasing effect resulting from light dispersion within a photoconductor layer (e.g., the first photoconductive layer 120 or the second photoconductive layer 130). By sufficiently shifting an energy baseline, a conductive path may be realized, and thus, it is possible to realize electrodes with polygonal shapes.

According to an embodiment, a CMOS-based device may be a monolithic integration device in which an electrode system on a CMOS chip is monolithically integrated with the CMOS chip. The CMOS-based device may be a heterogeneous integration device in which an electrode system on a CMOS chip is heterogeneously integrated with the CMOS chip.

According to an embodiment, the CMOS-based device may be an electrophysiological system-on-a-chip (SOC) for recording or stimulating signals from neurons or may be used to identify a neuronal system and evaluate a neuronal-based algorithm with a neuron-in-the-loop.

According to an embodiment, an electrode system integrated with a micro/nano-LED display, a CMOS-based device (e.g., an electrophysiological SOC), and an application device thereof may be manufactured using the standard semiconductor manufacturing process (e.g., patterning, deposition, planarization, etching, etc.) known in the art to which the disclosure belongs.

According to an embodiment, the disclosure may provide a CMOS-based device in which an electrode system with a nanometer-scale resolution to micrometer-scale resolution and addressability over a large area and an electrode system on a CMOS-compatible substrate are integrated. In some examples, the electrode system or the CMOS-based device may perform cell recording and stimulation based on a spatial reconfiguration of electrodes.

According to an embodiment, the electrode system 100 may provide light patterning calibration by a photoconductor (e.g., a second photoconductive layer) and the LED display layer 110 based on a fluorescence pattern emitted by biological cells.

According to an embodiment, the electrode system 100 may perform a light patterning process of setting a position or a range of a sample in which a task of an electrode is performed using at least one of a position of light emitted from the LED display layer 110, an area of light, a shape of a light spot or an intensity of light, based on the fluorescence pattern emitted by biological cells. Through the above light patterning, a position, a size, or a shape of an electrode may be reconfigured within the electrode layer 140 used for tasks (e.g., cell recording and stimulation).

According to an embodiment, the disclosure may provide a neuronal system identification based on a micro/nano-LED display coupled to a photoconductive layer.

According to an embodiment, the disclosure relates to a multi-channel electrode reconfigurable with a micro/nano-LED and a photoconductive metal, and a method of manufacturing the same. In some examples, a position, a size, or a shape of an electrode may be adjusted by on/off of a nano-LED array.

According to an embodiment, in the disclosure, a resolution may be increased in comparison to existing metal electrodes and electrophysiology may be measured at a desired position of a cell.

According to an embodiment, the disclosure may be associated with a micro/nano-LED display and a CMOS-based cell recording system to realize electrodes of which spatial distribution may be dynamically reconfigured.

According to an embodiment, in the disclosure, regions of neurons that are stimulated and recorded may be precisely selected after a cell growth, unlike the related art. This may be used to develop semiconductor devices and systems inspired by neuronal behaviors. For example, the CMOS chip may be configured to stimulate regions of neurons and record a response to the stimulation.

According to an embodiment, in the disclosure, a nano-scale resolution and a scalable addressable area, which is an inherent characteristic of micro- and nano-LED-based displays, may be enabled.

According to an embodiment, the disclosure may provide an electrode system and a CMOS-based device that enable temporary and reconfigurable patterning of electrodes and vias on a CMOS-compatible surface.

According to an embodiment, the disclosure may enable a precise selection of a neuron region to be recorded and excited after a cell growth. In some examples, a neuron-in-the-loop system may be designed to perform system identification of neuronal systems or to test neuronal-inspired algorithms in a biological environment.

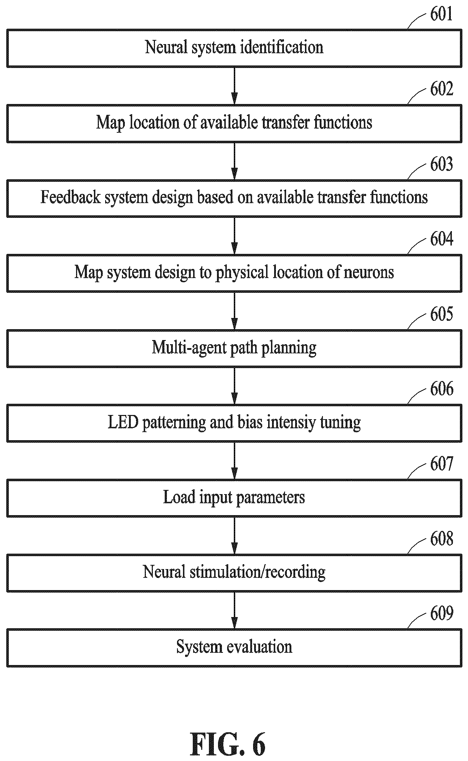

According to an embodiment, the electrode system 100 may accurately select a position of an electrode after a cell growth while being able to record thousands to millions of channels. As such, according to an embodiment, a system for identifying system characteristics of a neuronal network or a system for benchmarking performance of a neuronal-based algorithm using real neurons (FIG. 6), which is similar to a neuron-in-the-loop feedback system, may be provided.

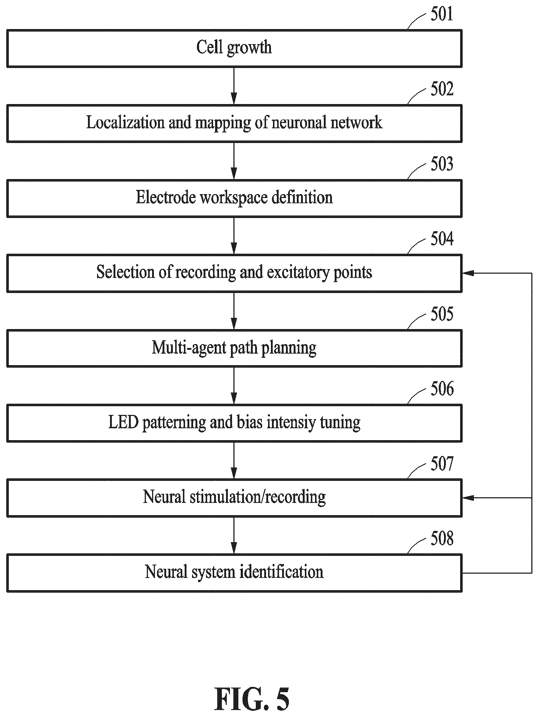

FIG. 5 is a flowchart illustrating a method of identifying a neuronal network or a neuronal system using a light-addressable electrode based on an LED display of an electrode system, according to one or more embodiments. FIG. 6 is a flowchart illustrating a method of evaluating a neuronal-based algorithm with a neuron-in-the-loop, using the method of FIG. 5 and a light-addressable electrode based on an LED display of an electrode system, according to one or more embodiments.

According to an embodiment, referring to FIGS. 5 and 6, a neuronal system identification process of FIG. 5 may include a cell growth (501), localization and mapping of a neuronal network (502), definition of an electrode workspace (503), selection of recording and excitatory points (504), multi-agent path planning (505), LED patterning and bias intensity tuning (506), neuronal stimulation and recording (507), and identifying of a neuronal system (508).

According to an embodiment, in FIG. 6, a process of evaluating neuronal-based algorithms may include identifying of a neuronal system (601), mapping of locations of available transfer functions (602), designing of a feedback system based on available transfer functions (603), mapping of a system design to physical locations of neurons (604), multi-agent path planning (605), LED patterning and bias intensity tuning (606), loading of input parameters (607), neuronal stimulation/recording (608), and a system evaluation (608). According to an embodiment, the multi-agent path planning may include properly routing vias and feedlines while avoiding short-circuit/crosstalk. In FIG. 6, after a cell growth and micro/nano-LED patterning (FIGS. 4A to 4E), which may include a multi-agent path planning to properly route vias and feedlines while avoiding short-circuit/crosstalk, I-V recordings (FIGS. 4A to 4E) from different regions may be iteratively performed (as part of an iterative loop of FIG. 5) until a system identification optimization procedure converges and a solution is found. As a result, system characteristics (i.e., neuronal transfer functions) of each individual neuron may be extracted, thereby enabling the design of artificial devices. In an example case in which system properties of a neuronal system are identified, the electrode distribution may be reconfigured to realize a feedback system with a neuron-in-the-loop, which may enable the evaluation of neuronal-based algorithms using neurons.

According to an embodiment, the disclosure may provide an integration of a cell recording system and a micro/nano-LED display.

According to an embodiment, the disclosure may provide a homogeneous or heterogeneous integration of micro/nano-LED displays to control conductivity of photoconductive layers for cell recording applications.

According to an embodiment, the disclosure may provide a neuron-in-the-loop feedback system realized using light-addressable electrodes.

According to an embodiment, the disclosure may provide a cell electroporation using light-addressable electrodes coupled to micro/nano-LED displays.

According to an embodiment, the disclosure may provide use of a micro/nano-LED display for a fluorescence microscopy of biological cells.

According to embodiments, the disclosure may provide a micro/nano-LED display-based reconfigurable electrode system and a CMOS-based MEA integrated therewith.

According to an embodiment, an electrode system may include an LED display layer, a first photoconductive layer on the LED display layer, a second photoconductive layer on the first photoconductive layer, and an electrode layer.

According to an embodiment, the LED display layer may include a nano-LED or a micro-LED.

According to an embodiment, an LED in the LED display layer may include at least one of a group II-VI compound semiconductor, a group III-V compound semiconductor or a group I-III-VI compound semiconductor, or a combination thereof.

According to an embodiment, the LED display layer may include a plurality of LEDs. The plurality of LEDs may be configured to form a plurality of pixel units by a single LED or a plurality of pixel units by an arrangement of the plurality of LEDs. The LED display layer may be configured to adjust a light emitting region, a shape of the light emitting region, or a light emission intensity within the plurality of pixel units. A spacing between the plurality of pixel units may be empty or filled with a photoconductive material.

According to an embodiment, the LED display layer may include a single layer or a plurality of layers. The electrode system may further include a nano-positioner or a micro-positioner configured to independently shift horizontally or vertically, or rotate each of a plurality of LED display layers in an example case in which the plurality of LED display layers is stacked.

In an example case in which a plurality of LED display layers is stacked, the nano-positioner or the micro-positioner may shift the LED display layers such that positions of pixel units of neighboring LED display layers overlap in a range of 0% to 100%, and shift the LED display layers such that light emitted from pixel units of neighboring LED display layers overlap.

According to an embodiment, a surface roughness of the first photoconductive layer may be different from a surface roughness of the second photoconductive layer.

According to an embodiment, the first photoconductive layer may include a flat film surface, and the second photoconductive layer may include a textured surface. The second photoconductive layer may include a textured surface with a 3D structure including at least one of a spherical shape, an oval shape, a conical shape, a polygonal pyramidal shape, a polygonal pillar shape, a polygonal shape, or a star polygonal shape, or a combination thereof.

According to an embodiment, the first photoconductive layer and the second photoconductive layer may each include at least one of a photoconductive polymer, a photoconductive chalcogenide, a photoconductive oxide or a photoconductive amorphous silicon-based material, or a combination thereof.

According to an embodiment, the electrode layer may include a plurality of electrode patterns on a support substrate, and the electrode patterns may each include at least one of a metal, a conductive oxide or a conductive polymer, or a combination thereof.

According to an embodiment, the electrode layer may include a plurality of electrode patterns that is at least partially inserted in a depth direction of a support substrate and that each has an exposed top surface.

According to an embodiment, the support substrate may be the first photoconductive layer.

According to an embodiment, the support substrate may be a transparent or opaque substrate.

According to an embodiment, a distance between the LED display layer and the second photoconductive layer may be in a range of about 0 nm (nanometer)(e.g., conformal deposition) to about 1 mm (millimeter)(e.g., heterogeneous integration).

According to an embodiment, the electrode system may be a monolithic integration system. The monolithic integration system may include an LED display layer, a first photoconductive layer on the LED display layer, and a second photoconductive layer on the first photoconductive layer. The monolithic integration system may further include an electrode layer in a lower region of the LED display layer. The electrode layer may include a support substrate, and an electrode pattern layer on the support substrate. The LED display layer may be in contact with the electrode pattern layer.

According to an embodiment, the electrode system may be a heterogeneous integration system. The heterogeneous integration system may include an LED display layer, a first photoconductive layer on the LED display layer, and a second photoconductive layer on the first photoconductive layer. The heterogeneous integration system may further include an electrode layer in an upper region of the LED display layer. The electrode layer may be provided on the second photoconductive layer. A plurality of LED display layers may be provided, and each of the LED layers may include a nano-positioner or a micro-positioner.

According to an embodiment, the electrode system may be configured to perform an imaging, a stimulation or a signal detection of a cell in vitro or in vivo. The electrode system may include a light-addressable electrode configured to set a position or a range of a sample in which a task of an electrode is performed using at least one of a position of light emitted from the LED display layer, an area of light, a shape of a light spot or a light intensity, and configured to reconfigure a position or a size of an electrode used for the task.

According to embodiments, the disclosure may provide a CMOS-based device manufactured through integration of a CMOS-based micro- and nano-LED display and a photoconductive layer.

According to an embodiment, a CMOS-based device may include a CMOS chip, and the electrode system according to an embodiment. The CMOS chip and the electrode system may be integrated.

According to an embodiment, the CMOS-based device may further include a bias configured to provide a light source to an upper end, a lower end, or both of the electrode system. The electrode system may be monolithically or heterogeneously integrated on the CMOS chip.

According to an embodiment, the CMOS-based device may be an electrophysiological SOC for stimulating a neuron or recording a signal from a neuron, or a CMOS-based cell recording device used to identify a neuronal system and evaluate a neuronal-based algorithm with a neuron in a loop.

According to an embodiment, the disclosure may provide a neuron-in-the-loop system that may be used to record and stimulate a neuronal activity for applications enabled by a reconfigurable neuron-in-the-loop feedback system, including system identification and evaluation of neuronal-based algorithms.

According to an embodiment, the disclosure may provide reconfigurable electrodes (e.g., light-addressable electrodes) that may reconfigure an electrode distribution with a sub-micron resolution while having an addressable area.

As described above, although the embodiments have been described with reference to the limited drawings, one of ordinary skill in the art may apply various technical modifications and variations based thereon. For example, suitable results may be achieved if the described techniques are performed in a different order, and/or if components in a described system, architecture, device, or circuit are combined in a different manner, and/or replaced or supplemented by other components or their equivalents.

The scope of the present invention is defined by the appended claims.

## Claims

1. An electrode system comprising:
a light-emitting diode (LED) display layer comprising a single LED layer (110) or a plurality of LED layers;
a first photoconductive layer (120) on the LED display layer;
a second photoconductive layer (130) on the first photoconductive layer; and
an electrode layer (140) provided on the LED display layer, the first photoconductive layer or the second photoconductive layer.

2. The electrode system of claim 1, wherein the LED display layer comprises a nano-LED or a micro-LED, and/or
wherein an LED in the LED display layer comprises at least one of a group II-VI compound semiconductor, a group III-V compound semiconductor or a group I-III-VI compound semiconductor, or a combination thereof.

3. The electrode system of claim 1 or 2,
wherein the LED display layer comprises a plurality of LEDs,
wherein the plurality of LEDs are configured to form a plurality of pixel units (111) based on a single LED, among the plurality of LEDs, or based on an arrangement of a plurality of LEDs,
wherein the plurality of LEDs are configured to adjust a light emitting region, a shape of the light emitting region, or a light emission intensity within the plurality of pixel units, and
wherein a spacing between the plurality of pixel units is empty or filled with a photoconductive material.

4. The electrode system of any of the preceding claims, further comprising:
a nano-positioner (160) or a micro-positioner (160) configured to independently shift horizontally or vertically, or rotate one or more of the plurality of LED layers, which are stacked,
in particular,
wherein the nano-positioner or the micro-positioner is further configured to:
shift the one or more of the plurality of LED layers such that positions of pixel units of neighboring LED layers overlap in a range of 0% to 100%; or
shift the one or more of the plurality of LED layers such that light emitted from pixel units of neighboring LED layers overlap.

5. The electrode system of any of the preceding claims, wherein a first surface roughness of the first photoconductive layer is different from a second surface roughness of the second photoconductive layer.

6. The electrode system of any of the preceding claims, wherein
the first photoconductive layer comprises a flat film surface, and
the second photoconductive layer comprises a textured surface with a three-dimensional (3D) structure comprising at least one of a spherical shape, an oval shape, a conical shape, a polygonal pyramidal shape, a polygonal pillar shape, a polygonal shape, or a star polygonal shape, or a combination thereof.

7. The electrode system of any of the preceding claims, wherein
each of the first photoconductive layer and the second photoconductive layer comprise at least one of a photoconductive polymer, a photoconductive chalcogenide, a photoconductive oxide or a photoconductive amorphous silicon-based material, or a combination thereof.

8. The electrode system of any of the preceding claims, wherein
the electrode layer comprises a plurality of electrode patterns on a support substrate, and
each of the of the plurality of electrode patterns comprise at least one of a metal, a conductive oxide or a conductive polymer, or a combination thereof,
in particular,
wherein the electrode layer comprises a plurality of electrode patterns that is at least partially inserted in a depth direction of a support substrate (150) and each of the plurality of electrode patterns has an exposed top surface.

9. The electrode system of any of the preceding claims, wherein the support substrate is the first photoconductive layer, and/or
wherein the support substrate is a transparent substrate or opaque substrate.

10. The electrode system of any of the preceding claims, wherein a distance between the LED display layer and the second photoconductive layer is in a range of 1 nm to 1,000 nm.

11. The electrode system of any of the preceding claims, wherein the electrode system is a monolithic integration system in which the electrode layer is provided on a lower region of the LED display layer, and
wherein the electrode layer comprises:
a support substrate; and
an electrode pattern layer on the support substrate, and
wherein the LED display layer is in contact with the electrode pattern layer.

12. The electrode system of any of the preceding claims, wherein the electrode system is a heterogeneous integration system in which the electrode layer is provided on an upper region of the LED display layer and on the second photoconductive layer, and
wherein each of the plurality of LED layers comprises a nano-positioner or a micro-positioner.

13. The electrode system of any of the preceding claims, wherein
the electrode system is configured to perform an imaging, a stimulation or a signal detection of a cell in vitro or in vivo, and
the electrode system comprises a light-addressable electrode provided in the electrode layer, the light-addressable electrode configured to:
set a position or a range of a sample based on at least one of a position of light emitted from the LED display layer, an area of light, a shape of a light spot or a light intensity, and
reconfigure a position or a size of the light-addressable electrode.

14. A complementary metal-oxide-semiconductor (CMOS)-based device comprising:
a CMOS chip; and
an electrode system comprising according to any of the preceding claims.

15. The CMOS-based device of claim 14, wherein
the CMOS-based device further comprises a bias configured to provide a light source to an upper end, a lower end, or both the upper end and the lower end of the electrode system, and
the electrode system is monolithically or heterogeneously integrated on the CMOS chip,
in particular,
wherein the CMOS-based device is an electrophysiological system-on-a-chip (SOC) for recording or stimulating a signal from a neuron, or a CMOS-based cell recording device used to identify a neuronal system and evaluate a neuronal-based algorithm with a neuron in a loop.

## Patentansprüche

1. Elektrodensystem, das umfasst:
eine LED-Anzeigeschicht, die eine einzelne LED-Schicht (110) oder eine Vielzahl von LED-Schichten umfasst;
eine erste photoleitende Schicht (120) auf der LED-Anzeigeschicht;
eine zweite photoleitende Schicht (130) auf der ersten photoleitenden Schicht; sowie
eine Elektrodenschicht (140), die sich auf der LED-Anzeigeschicht der ersten photoleitenden Schicht oder der zweiten photoleitenden Schicht befindet.

2. Elektrodensystem nach Anspruch 1, wobei die LED-Anzeigeschicht eine Nano-LED oder
eine Mikro-LED umfasst, und/oder
wobei eine LED in der LED-Anzeigeschicht einen Gruppe-II-VI-Verbindungshalbleiter, einen Gruppe-III-V-Verbindungshalbleiter oder/und einen Gruppe-I-III-VI-Verbindungshalbleiter oder eine Kombination derselben umfasst.

3. Elektrodensystem nach Anspruch 1 oder 2,
wobei die LED-Anzeigeschicht eine Vielzahl von LED umfasst,
wobei die Vielzahl von LED so konfiguriert sind, dass sie eine Vielzahl von Pixel-Einheiten basierend auf einer einzelnen LED unter der Vielzahl von LED oder basierend auf einer Anordnung einer Vielzahl von LED bilden,
wobei die Vielzahl von LED so konfiguriert sind, dass sie einen lichtemittierenden Bereich, eine Form des lichtemittierenden Bereiches oder eine Lichtemissions-Intensität innerhalb der Vielzahl von Pixel-Einheiten regulieren, und
wobei ein Zwischenraum zwischen der Vielzahl von Pixel-Einheiten leer ist oder mit einem photoleitenden Material gefüllt ist.

4. Elektrodensystem nach einem der vorangehenden Ansprüche, das des Weiteren umfasst:
einen Nanopositionierer (160) oder einen Mikropositionierer (160), der so konfiguriert ist, dass er eine oder mehrere der Vielzahl von LED-Schichten, die übereinandergeschichtet sind, unabhängig horizontal oder vertikal verschiebt oder rotiert,
wobei insbesondere der Nanopositionierer oder der Mikropositionierer des Weiteren konfiguriert ist zum:
Verschieben der einen oder mehreren der Vielzahl von LED-Schichten so, dass Positionen von Pixel-Einheiten benachbarter LED-Schichten einander in einem Bereich von 0 % bis 100 % überlappen; oder
Verschieben der einen oder mehreren der Vielzahl von LED-Schichten so, dass es zu Überlappung von Pixel-Einheiten benachbarter LED-Schichten emittierten Lichtes kommt.

5. Elektrodensystem nach einem der vorangehenden Ansprüche, wobei eine erste Oberflächenrauigkeit der ersten photoleitenden Schicht sich von einer Oberflächenrauigkeit der zweiten photoleitenden Schicht unterscheidet.

6. Elektrodensystem nach einem der vorangehenden Ansprüche, wobei
die erste photoleitende Schicht eine flache Filmfläche umfasst, und
die zweite photoleitende Schicht eine texturierte Fläche mit einer dreidimensionalen Struktur umfasst, die eine sphärische Form, eine ovale Form, eine konische Form, eine polygonale Pyramidenform, eine polygonale Säulenform, eine polygonale Form oder/und eine polygonale Sternform oder eine Kombination derselben umfasst.

7. Elektrodensystem nach einem der vorangehenden Ansprüche, wobei
die erste photoleitende Schicht und die zweite photoleitende Schicht jeweils ein photoleitendes Polymer, ein photoleitendes Chalkogenid, ein photoleitendes Oxid oder/und ein photoleitendes amorphes Material auf Siliziumbasis oder eine Kombination derselben umfassen.

8. Elektrodensystem nach einem der vorangehenden Ansprüche, wobei
die Elektrodenschicht eine Vielzahl von Elektrodenmustern auf einem Trägersubstrat umfasst, und
jedes der Vielzahl von Elektrodenmustern ein Metall, ein leitendes Oxid oder/und ein leitendes Polymer oder eine Kombination derselben umfasst,
und wobei insbesondere die Elektrodenschicht eine Vielzahl von Elektrodenmustern umfasst, die wenigstens teilweise in einer Tiefenrichtung eines Trägersubstrats (150) eingefügt sind,
und jedes der Vielzahl von Elektrodenmustern eine freigelegte obere Fläche aufweist.

9. Elektrodensystem nach einem der vorangehenden Ansprüche, wobei das Trägersubstrat die erste photoleitende Schicht ist und/oder
wobei das Trägersubstrat ein transparentes Substrat oder ein opakes Substrat ist.

10. Elektrodensystem nach einem der vorangehenden Ansprüche, wobei ein Abstand zwischen der LED-Anzeigeschicht und der zweiten photoleitenden Schicht in einem Bereich von 1 nm bis 1.000 nm liegt.

11. Elektrodensystem nach einem der vorangehenden Ansprüche, wobei das Elektrodensystem ein monolithisches Integrationssystem ist, in dem die Elektrodenschicht sich in einem unteren Bereich der LED-Anzeigeschicht befindet, und
wobei die Elektrodenschicht umfasst:
ein Trägersubstrat; sowie
eine Elektrodenmuster-Schicht auf dem Trägersubstrat, und
wobei die LED-Anzeigeschicht in Kontakt mit der Elektrodenmuster-Schicht ist.

12. Elektrodensystem nach einem der vorangehenden Ansprüche, wobei das Elektrodensystem ein heterogenes Integrationssystem ist, in dem die Elektrodenschicht sich in einem oberen Bereich der LED-Anzeigeschicht sowie auf der zweiten photoleitenden Schicht befindet, und
wobei jede der Vielzahl von LED-Schichten einen Nanopositionierer oder einen Mikropositionierer umfasst.

13. Elektrodensystem nach einem der vorangehenden Ansprüche, wobei
das Elektrodensystem zum Durchführen einer Bilderzeugung, einer Stimulation oder einer Signalerfassung einer Zelle in vitro oder in vivo konfiguriert ist, und
das Elektrodensystem eine lichtadressierbare Elektrode umfasst, die sich in der Elektrodenschicht befindet, wobei die lichtadressierbare Elektrode konfiguriert ist zum:
Festlegen einer Position oder eines Bereiches einer Probe auf Basis einer Position von der LED-Anzeigeschicht emittierten Lichtes, einer Lichtfläche, einer Form eines Lichtpunktes oder/und einer Lichtintensität, sowie
Rekonfigurieren einer Position oder einer Größe der lichtadressierbaren Elektrode.

14. Vorrichtung auf Basis eines komplementären Metalloxid-Halbleiters (CMOS), die umfasst:
einen CMOS-Chip; sowie
ein Elektrodensystem nach einem der vorangehenden Ansprüche.

15. Vorrichtung auf Basis eines komplementären Metalloxid-Halbleiters nach Anspruch 14, wobei
die Vorrichtung auf Basis eines komplementären Metalloxid-Halbleiters des Weiteren eine Vorspannung umfasst, die so konfiguriert ist, dass sie eine Lichtquelle an einem oberen Ende, einem unteren Ende oder sowohl dem oberen Ende als auch dem unteren Ende des Elektrodesystems bildet, und
das Elektrodensystem monolithisch oder heterogen auf dem CMOS-Chip integriert ist,
wobei insbesondere die Vorrichtung auf Basis eines komplementären Metalloxid-Halbleiters ein elektrophysiologisches System-on-a-Chip (SOC) zum Aufzeichnen oder Stimulieren eines Signals von einem Neuron oder ein CMOS-basiertes Zell-Registriergerät ist, das zum Identifizieren eines neuronalen Systems sowie zum Evaluieren eines neuronal-basierten Algorithmus mit einem Neuron in einer Schleife dient.

## Revendications

1. Système d'électrode comprenant:
une couche d'affichage à diodes électroluminescentes (LED) comprenant une seule couche de LED (110) ou une pluralité de couches de LED;
une première couche photoconductrice (120) sur la couche d'affichage à LED;
une seconde couche photoconductrice (130) sur la première couche photoconductrice; et
une couche d'électrode (140) disposée sur la couche d'affichage à LED, la première couche photoconductrice ou la seconde couche photoconductrice.

2. Système d'électrode selon la revendication 1, dans lequel la couche d'affichage à LED comprend une nano-LED ou une micro-LED, et/ou
dans lequel une LED de la couche d'affichage à LED comprend au moins un semi-conducteur composé du groupe II-VI, un semi-conducteur composé du groupe III-V ou un semi-conducteur composé du groupe I-III-VI, ou une combinaison de ceux-ci.

3. Système d'électrode selon les revendications 1 ou 2,
dans lequel la couche d'affichage à LED comprend une pluralité de LED,
dans lequel la pluralité de LED est configurée pour former une pluralité d'unités de pixels (131) sur la base d'une seule LED parmi la pluralité de LED, ou sur la base d'un agencement d'une pluralité de LED,
dans lequel la pluralité de LED est configurée pour ajuster une zone d'émission de lumière, une forme de la zone d'émission de lumière ou une intensité d'émission de lumière au sein de la pluralité d'unités de pixels, et
dans lequel un espace entre la pluralité d'unités de pixels est vide ou rempli d'un matériau photoconducteur.

4. Système d'électrode selon l'une quelconque des revendications précédentes, comprenant en outre:
un nanopositionneur (160) ou un micropositionneur (160) configuré pour déplacer indépendamment horizontalement ou verticalement, ou faire tourner une ou plusieurs des multiples couches de LED, qui sont empilées,
en particulier,
dans lequel le nano-positionneur ou le micro-positionneur est en outre configuré pour:
déplacer une ou plusieurs des couches de LED de telle sorte que les positions des unités de pixels de couches de LED voisines se chevauchent dans une plage comprise entre 0 % et 100 %; ou bien
déplacer une ou plusieurs des couches de LED de la pluralité de couches de LED de telle sorte que la lumière émise par les unités de pixels de couches de LED voisines se chevauche.

5. Système d'électrode selon l'une quelconque des revendications précédentes, dans lequel une première rugosité de surface de la première couche photoconductrice est différente d'une seconde rugosité de surface de la seconde couche photoconductrice.

6. Système d'électrode selon l'une quelconque des revendications précédentes, dans lequel
la première couche photoconductrice comprend une surface de film plane, et
la seconde couche photoconductrice comprend une surface texturée présentant une structure tridimensionnelle (3D) comprenant au moins l'une des formes suivantes: une forme sphérique, une forme ovale, une forme conique, une forme pyramidale polygonale, une forme de pilier polygonal, une forme polygonale ou une forme polygonale en étoile, ou une combinaison de celles-ci.

7. Système d'électrode selon l'une quelconque des revendications précédentes, dans lequel
la première couche photoconductrice et la seconde couche photoconductrice comprennent chacune au moins un polymère photoconducteur, un chalcogénure photoconducteur, un oxyde photoconducteur ou un matériau photoconducteur à base de silicium amorphe, ou une combinaison de ceux-ci.

8. Système d'électrode selon l'une quelconque des revendications précédentes, dans lequel
la couche d'électrode comprend une pluralité de motifs d'électrodes sur un substrat de support, et
chacun des motifs d'électrode de la pluralité comprend au moins un élément parmi un métal, un oxyde conducteur ou un polymère conducteur, ou une combinaison de ceux-ci,
en particulier,
dans lequel la couche d'électrode comprend une pluralité de motifs d'électrodes qui sont au moins partiellement insérés dans le sens de la profondeur d'un substrat de support (150) et chacun des motifs d'électrodes de la pluralité présente une surface supérieure exposée.

9. Système d'électrode selon l'une quelconque des revendications précédentes, dans lequel le substrat de support est la première couche photoconductrice, et/ou
dans lequel le substrat de support est un substrat transparent ou un substrat opaque.

10. Système d'électrode selon l'une quelconque des revendications précédentes, dans lequel la distance entre la couche d'affichage à LED et la deuxième couche photoconductrice est comprise dans une plage de 1 nm à 1 000 nm.

11. Système d'électrode selon l'une quelconque des revendications précédentes, dans lequel le système d'électrode est un système d'intégration monolithique dans lequel la couche d'électrode est prévue sur une zone inférieure de la couche d'affichage à LED, et
dans lequel la couche d'électrode comprend:
un substrat de support; et
une couche de motif d'électrode sur le substrat de support, et
dans lequel la couche d'affichage à LED est en contact avec la couche de motifs d'électrodes.

12. Système d'électrode selon l'une quelconque des revendications précédentes, dans lequel le système d'électrode est un système d'intégration hétérogène dans lequel la couche d'électrode est disposée sur une zone supérieure de la couche d'affichage à LED et sur la seconde couche photoconductrice, et
dans lequel chacune des multiples couches de LED comprend un nanopositionneur ou un micropositionneur.

13. Système d'électrode selon l'une quelconque des revendications précédentes, dans lequel
le système d'électrode est configuré pour effectuer une imagerie, une stimulation ou une détection de signal d'une cellule in vitro ou in vivo, et
le système d'électrode comprend une électrode adressable par la lumière prévue dans la couche d'électrode, l'électrode adressable par la lumière étant configurée pour:
définir une position ou une plage d'un échantillon sur la base d'au moins l'un des éléments suivants: une position de la lumière émise par la couche d'affichage à LED, une zone de lumière, une forme d'un spot lumineux ou une intensité lumineuse, et
reconfigurer la position ou la taille de l'électrode adressable par la lumière.

14. Dispositif à semi-conducteurs à oxyde métallique complémentaire (CMOS) comprenant:
une puce CMOS; et
un système d'électrode comprenant l'un quelconque des éléments selon les revendications précédentes.

15. Dispositif à base de CMOS selon la revendication 14, dans lequel
le dispositif à base de CMOS comprend en outre une polarisation configurée pour fournir une source lumineuse à une extrémité supérieure, à une extrémité inférieure, ou à la fois à l'extrémité supérieure et à l'extrémité inférieure du système d'électrode, et
le système d'électrode est intégré de manière monolithique ou hétérogène sur la puce CMOS,
en particulier,
dans lequel le dispositif à base de CMOS est un système électrophysiologique sur puce (SOC) destiné à enregistrer ou à stimuler un signal provenant d'un neurone, ou un dispositif d'enregistrement cellulaire à base de CMOS utilisé pour identifier un système neuronal et évaluer un algorithme neuronal avec un neurone en boucle.
